# EUROPEAN PATENT SPECIFICATION

(11) **EP 2 620 494 B1**
(45) Date of publication and mention of the grant of the patent: **22.02.2017**
(21) Application number: 11787917.1
(22) Date of filing: 20.09.2011
(51) Int. Cl.: C12N 9/04, C12P 7/62, C12P 11/00, C12R 1/40, C07C 319/20, C07C 323/52, C07C 327/32, C08G 63/78

(54) **SYNTHESIS OF POLYHYDROXYALKANOATES (PHA) WITH THIOESTER GROUPS IN THE SIDE CHAIN**
SYNTHESE VON POLYHYDROXYALKANOATEN MIT THIOESTER-GRUPPEN IN DER SEITENKETTE
SYNTHÈSE DE POLYHYDROXYALCANOATES (PHA) COMPORTANT DES GROUPES THIOESTERS DANS LA CHAÎNE LATÉRALE

(30) Priority: 21.09.2010 ES 201031401
(43) Date of publication of application: 31.07.2013
(73) Proprietor: Consejo Superior De Investigaciones Científicas (CSIC), 28006 Madrid (ES)
(72) Inventor: FERNÁNDEZ ESCAPA, Isabel, E-28040 Madrid (ES); MORALES RUIZ, Mª del Valle, E-28040 Madrid (ES); GARCÍA LÓPEZ, José Luís, E-28040 Madrid (ES); PRIETO JIMÉNEZ, Mª Auxiliadora, E-28040 Madrid (ES)
(74) Representative: Pons
(86) International application number: PCT/ES2011/070654
(87) International publication number: WO 2012/038572

(56) References cited:
- WO-A2-02/16627
- SHAO-PING OUYANG ET AL: "Production of Polyhydroxyalkanoates with High 3-Hydroxydodecanoate Monomer Content by fadB and fadA Knockout Mutant of Pseudomonas putida KT2442", BIOMACROMOLECULES, vol. 8, no. 8, 1 August 2007 (2007-08-01), pages 2504-2511, XP55019220, ISSN: 1525-7797, DOI: 10.1021/bm0702307
- ISABEL F ESCAPA ET AL: "Disruption of Î-oxidation pathway inKT2442 to produce new functionalized PHAs with thioester groups", APPLIED MICROBIOLOGY AND BIOTECHNOLOGY, SPRINGER, BERLIN, DE, vol. 89, no. 5, 26 January 2011 (2011-01-26), pages 1583-1598, XP019880838, ISSN: 1432-0614, DOI: 10.1007/S00253-011-3099-4

### SEQUENCE LISTING

<110> Consejo Superior de Investigaciones Cientificas
<120> SYNTHESIS OF POLYHYDROXYALKANOATES (PHA) WITH THIOESTER GROUPS IN THE SIDE CHAIN
<130> PCT1641.792
<150> ES201031401
   <151> 2010-09-21
<160> 2
<170> PatentIn version 3.5
<210> 1
   <211> 31
   <212> DNA
   <213> Pseudomonas putida
<400> 1
   tcccccggga aaagctcaag ctcaatgcca t 31
<210> 2
   <211> 26
   <212> DNA
   <213> Pseudomonas putida
<400> 2
   tcccccgggt tgaagaagtg catgcc 26

## Description

The present invention relates to a novel process for the synthesis of polyhydroxyalkanoates (PHAs) and PHAs obtained by the said process. Furthermore, the present invention relates to the use of the said PHA. Therefore, the present invention pertains to the fields of Environmental Biotechnology, Microbiology and Biopolymers.

### STATE OF THE ART

Polyhydroxyalkanoates (PHA), commonly known as "bioplastics" are biodegradable polymers produced by certain bacteria, which accumulate inside the cell in the form of carbon source storage granules when growing conditions are not optimal for growth (Madison and Huisman, 1999. Microbiol. Mol. Biol Rev., 63: 21-53). These biopolymers are biodegradable and are synthesized by bacteria from renewable sources such as, for example, glucose, fructose or fatty acids that make up a portion of vegetable oils. Therefore, the term bioplastic can be defined as a biopolymer synthesized from renewable sources, which can be biodegraded under controlled conditions and presents physicochemical characteristics similar to those of plastics derived from the Petrochemical industry (Sarasa et al., 2009. Bioresour. Technol. 100: 3764-3768).

In general, PHA granules are composed of a polyester (93-97% of granule dry weight (GDW)) encircled by phospholipids (1-6% of GDW) and granule-associated proteins (GAPs) (1-2% of GDW), which form a thin layer on the granule surface. To date, three types of GAP have been defined in bacteria: i) PHA synthases, involved in PHA polymerization; ii) PHA depolymerases, responsible for bioplastic degradation; and iii) phasins, the most abundant GAPs, with a structural or regulatory function Prieto et al., 1999a. Appl. Environ. Microbiol., 65: 3265-3271; Moldes et al., 2004. Appl. Environ. Microbiol., 70: 3205-3212).

PHAs are classified into two main types according to their chemical structure: the short-chain-length PHAs (scl-PHAs) obtained from monomers with 4 or 5 carbon atoms, and the medium-chain-length (mcl-PHAs) obtained from monomers with 6 to 14 carbon atoms. The different PHAs identified to date are lineal polymers composed of 3-hydroxy fatty acids exclusively of the R configuration (RHAs). The molecular weight of these polymers varies between 50,000 and 1,000,000 and their diversity lies in the substitutions of the asymmetric carbon in position 3, which confers the chiral polymer characteristic (Prieto et al., (1999b) J. Bacteriol. 181: 858-868).

Polymer composition is known to depend on the carbon source present in the culture medium used during fermentation of the producer strain (Durner et al., 2001. Biotechnol. Bioeng., 72: 278-288, Jung et al. 2001. Biotechnol. Bioeng., 72: 19-24). Furthermore, it is important to note that the physicochemical characteristics of polymers vary depending on the chemical nature of the monomers that compose them (Madison and Huisman, 1999. Microbiol. Mol. Biol Rev., 63: 21-53, Kessler et al., 2001. J. Biotechnol., 86: 97-104). More than 140 different RHAs have been described as components of bacterially produced PHAs (Steinbüchel et al., 1995. Can. J. Microbiol., 41: 94-105; Sudesh et al., 2000. Prog. Polym. Sci, 25: 1503-1555), moreover after production by fermentation, the biopolymer can be subjected to further chemical modifications, such as crosslinking and addition of functional groups (Lageveen R.G. et al., 1988. Appl. Environ. Microbiol., 54: 2924-2932); therefore, a wide variety of bioplastics and different RHAs may potentially be generated by a combination of all these processes. It is also important to note that PHAs may also be useful as biomaterials for biomedical applications (Zinn et al., 2001. Adv. Drug. Deliv. Rev., 53: 5-21). In addition, PHA can be considered as a source of new chiral compounds (synthons), which are very useful as precursors in the pharmaceutical industry, because they are difficult to obtain in the pure state by conventional chemical processes.

Seeing that PHAs are non-toxic, biodegradable and biocompatible bacterial bio-polyesters produced by a wide range of bacteria, including *Pseudomonas (P*.*)*, these compounds have great potential in biomedical and pharmaceutical applications, due to their potential use as biomaterials and their application in controlled drug delivery systems and tissue engineering. PHAs have also attracted considerable attention due to their potential use as sources of chiral or synthon monomers, which are highly useful as precursors in the pharmaceutical industry because they are difficult to obtain in pure form by conventional chemical processes. The physicochemical properties of PHAs range from rigid and crystalline to flexible, amorphous and elastomeric, depending on their monomer composition and the length of the side group in the polymer.

Many PHAs have been described with functional groups in their side chains, such as bromine, chlorine, fluorine, a branched alkyl group, a cyano group, a hydroxyl group, an alkoxy group, an acetoxy group, an epoxy group, a cyclohexyl group, phenylthio group and a propylthio group (Kim et al., 2007. The Journal of Microbiology. 45, 2, 87-97). The latter two are the only examples of PHAs containing sulphur in the side chain. PHAs containing a thiophenoxy group in the side chain were obtained from *P. putida* 27N01 using 11-thiophenoxy-undecanoic acid as sole carbon source (Takagi et al., 1999. Macromolecules 32, 8315-18). PHAs containing a propylthio group in the side chain are composed of hydroxyl-propyl-thioalkanoic acid monomers. PHAs have also been synthesized containing thioethers in the side chain using a recombinant strain of *Ralstonia eutropha,* mutant for polyhydroxybutyrate production and for expression of the enzyme PHA synthase of *P. mendocina,* used as a propyl-thio-octanoic acid or propyl-thio-hexanoic acid substrate (Ewering et al., 2002. Microbiology 148, 1397-1406). This strain synthesizes from propyl-thio-octanoic acid, the terpolyester composed of 3-hydroxy-propyl-thio-butanoic acid, 3-hydroxy-propyl-thio-hexanoic acid and 3-hydroxy-propyl-thio-octanoic acid, and from propyl-thio-haxanoic acid, it synthesizes polyester of 3-hydroxy-propyl-thio-butanoic and 3-hydroxy-propyl-thio-hexanoic acid. All these PHAs contain thioether groups in the side chains; however, to date PHAs containing side-chain thioesters are unknown.

The document by Shao-Ping Ouyang et al. Biomacromolecules; August 2007, vol. 8 (8) describes the production of medium-chain-length PHAs by *Pseudomonas putida KT2442.*

The document WO02/16627 discloses the production of PHA by fermentation of bacteria such as *Pseudomonas putida* with appropriate sulphur containing substrates.

The synthesis of novel PHAs is still of great interest as new biopolymers, as well as their component monomers, with new physicochemical properties are potentially extremely useful for industry.

### DESCRIPTION OF THE INVENTION

The present invention relates to a polyhydroxyalkanoates (PHA) synthesis process, it also relates to the PHAs obtained by this process, and to the use of the said PHAs. The PHAs of the present invention, called PHACOS, have a new monomeric composition, as they contain monomers containing thioester groups in the side chain that confer new physicochemical properties, such as for example (i) a high glass transition temperature compared to conventional PHA, (ii) they do not have a melting point, as they are completely amorphous, and (iii) subsequent chemical modification is possible. The thioester group in the side chain of PHACOS affords the advantage of facilitating chemical modification, which implies that the modified PHA will have some new properties, conferring new applications and/or improvements to them. For example, the thioester group of the side chain enables PHACOS to bind to peptides and other molecules.

PHACOS monomers are *(R)-*3-hydroxy-acylthioalkanoic acids, such as *(R)*-3-hydroxy-6-acetylthiohexanoic acid or *(R)*-3-hydroxy-4-acetylthiobutanoic acid. These monomers can easily be chemically modified and, therefore, they are highly valuable for use in the pharmaceutical industry as synthons. The present invention also describes a process to obtain these enantiopure *(R)*-3-hydroxy-acylthioalkanoates from PHACOS.

Therefore, a first aspect of the present invention relates to a process for the synthesis of polyhydroxyalkanoates (PHAs) comprising monomers containing thioester groups in the side chain, which involves culturing a cell capable of producing PHA which is a bacterium of the genus *Pseudomonas* in a substrate, where the said substrate comprises at least one acylthioalkanoic acid and at least a fatty acid of between 4 and 28 carbons not functionalized with a thioester group.

The plant cell or the yeast can be genetically modified, i.e. they may be recombinant cells.

A bacterium is an organism whose species belongs to the Kingdom *Bacteria.* A bacterium pertaining to the Genus *Pseudomonas,* belonging to the Family *Pseudomonadaceae,* to the Order *Pseudomonadales,* to the Class *Gammaproteobacteria,* to the Phylum *Proteobacteria* and to the Kingdom *Bacteria.*

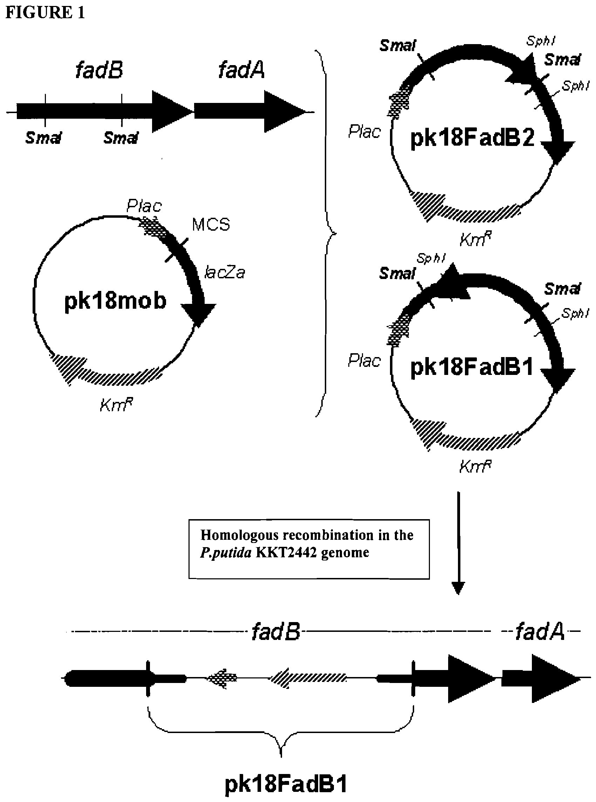

In a preferred embodiment of the first aspect of the invention, the bacteria of the genus *Pseudomonas* is selected from the list that comprises: *P. putida* KT2442, *P. putida* U, *P. oleovorans* GPo1, *P. putida* KT42FadB and *P. putida* U ΔFadBA. Preferably, the bacterium is *P. putida* KT42FadB. The strain *P*. *putida* KT42FadB was obtained by the authors of the present invention from the strain *P. putida* KT2442 as disclosed in Example 1 and Figure 1 of this report. In addition, the strain *P. putida* KT42FadB is deposited in the Spanish Type Culture Collection (Coleccion Española de Cultivos Tipo - CECT) with accession number CECT 7772.

The term "substrate", as used in this description, relates to the compound or compounds that the cell will use as a reagent or reagents to synthesize PHAs.

An "aliphatic compound" is an organic molecule composed of carbon and hydrogen, where C-C bonds can be single, double or triple. Preferably, it is a linear and/or branched organic molecule of C₅-C₁₄. A "functionalized aliphatic compound" is one in which at least one hydrogen is replaced by a functional group. A "functional group" is a group of specific atoms within a molecule that is responsible for the chemical reaction or reactions that characterize the said molecule. Examples of functional groups are: an ester group, a phenol group, a hydroxyl group or an amino group. Numerous carbon substrates have been described containing functionalized groups and acting as reagents in the synthesis of bacterial PHAs, but none with a thioester group. In addition, monomers that comprise a PHA are determined by the substrates used in their synthesis, and to date more than 140 hydroxyalkanoates have been described (Kim et al., 2007. The Journal of Microbiology. 45; 2: 87-97). The PHA monomers described to date have highly varied structures: linear, branched, saturated, unsaturated and aromatic. The presence of functionalized groups is of particular interest since this provides a variety of important physicochemical properties, for example, mechanical ones, and besides, some of these groups permit chemical modifications. Furthermore, since the direct use of these polyesters is difficult due, mainly, to their hydrophobic nature, the presence of hydrophilic groups in the PHA allows the production of amphiphilic polymers capable of forming micelles. The amphiphilic block copolymers are potential thickeners, emulsifiers, dispersants or foamers.

A thioester is a compound with a thioester functional group, which is represented as follows:

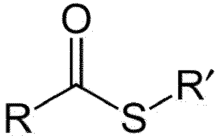

Where R and R' are independently two identical or different organic groups. A thioester is formed by the esterification between a carboxylic acid and a thiol.

Preferably the acylthioalkanoic acid is selected from the list that comprises the following acids: 6-acetylthiohexanoic acid, 5-acetylthiopentanoic acid, 2,5-bis(acetylsulfanyl)pentanoic acid, 4-acetylthiolbutanoic, 3-acetylsulfanylbutanoic, 11-acetylthioundecanoic, 16-acetylthiolhexadecanoic, 5-acetylthio-octanoic, 5-propane-ylthio-octanoic, 5-butane-ylthio-octanoic, 12-acetyl-thio-dodecanoic, 6-acetylsulfanyl-8-methylsulfanyl-octanoic, 8-acetylsulfanyl-6-sulfanyloctanoic, 5-(2-methylpropane-yl-sulfanyl)octane, 6,8-bis(acetylsulfanyl) octanoic. Preferably, the functionalized aliphatic compound is acid 6-acetyl-thiohexanoic acid (6-ATH).

In a preferred embodiment the fatty acid of between 4 and 28 carbons, may or may not have another type of functional group. This fatty acid is capable of inducing PHA synthesis, thus improving culture efficiency and the ability of cells to grow and synthesize PHA. The fatty acid may have single bonds and can also have double or triple bonds. The fatty acid can also be any of the group A substrates described by Kim et al., 2007. The Journal of Microbiology. 45, 2, 87-97. Preferably, the fatty acid is selected from the list that comprises decanoic acid, nonanoic acid and octanoic acid. It is even more preferable if the aliphatic compound is decanoic acid.

The term "inducer" as used herein, refers to the ability of a substrate to improve PHA synthesis.

In a preferred embodiment of the first aspect of the invention, the substrate comprises decanoic acid and 6-acetylthiohexanoic acid. These compounds may be present in different proportions. In a preferred embodiment of the first aspect of the invention, the substrate comprises a molar ratio of the fatty acid of between 4 and 28 carbons and the acylthioalkanoic acid of between 1:9 and 9:1. Preferably, the ratio is between 1.1:8.9 and 2:8. It is even more preferable if it is between 1.5:8.5 and 1.8:8.2. In a more preferred embodiment of the first aspect of the invention, the substrate consists of 2.4 mM decanoic acid and 12 mM 6-acetylthiohexanoic acid.

In a preferred embodiment of the first aspect of the invention, the culture comprises one or two fermentation stages. Preferably, it comprises two-stage fermentation. Preferably, the second fermentation stage lasts between 5 and 30 hours. More preferably, the duration is between 7 and 26 hours. Even more preferably, the duration is between 8 and 25 hours. In the most preferred embodiment, the second stage lasts 24 hours.

A "fermentation stage" relates to the culture taking place maintaining the same culture conditions throughout fermentation, with the same medium and at the same temperature. When cells are cultured in two-stage fermentation, different culture conditions are employed in the first fermentation stage and in the second fermentation stage. It is also understood that in cultures made in two-stage fermentation, either part of the culture or the whole culture produced in the first stage of fermentation is used as culture inoculum for the second stage of fermentation.

A second aspect of the invention relates to the PHAs obtained using the procedure described in the first aspect of the invention. These PHAs are hereinafter referred to as *PHACOS.*

A third aspect of the invention relates to the bacterial strain of the species *Pseudomonas putida* with accession number CECT 7772. The strain *P*. *putida* KT42FadB was deposited in the Spanish Type Culture Collection (CECT) on July 21, 2010 and has the accession number CECT 7772. The address of the international depositary authority is: Colección Española de Cultivos Tipo (CECT) / Universidad de ValenciaParc Científic Universitat de València / Catedrático Agustín Escardino, 9 / 46980 Paterna (Valencia).

The characteristics of the said strain are:
- It grows in LB rich medium ("Luria Bertani" or "Lysogen Broth", medium number 48 on the CECT media list, whose composition is 10 g/L tryptone, 5 g/L yeast extract and 10 g/L NaCl in water, pH 7.0) supplemented with kanamycin 50 µg/mL.
- It grows under orbital shaking at 300 rpm and at 30 °C for 14 hours.
- It is stored at -80 °C with glycerol at 15%.
- It is a disruptional insertion mutant with pK18*mob* plasmid of the *fadB* gene (PP_2136). The said plasmid has a gene for resistance to kanamycin.

A fourth aspect of the invention relates to the use of PHACOS to produce bioplastic materials, fabrics, biomaterials, drug delivery systems, adhesives or paints.

A fifth aspect of the invention relates to the use of PHACOS to obtain enantiopure *(R)*-3-hydroxy-acylthioalkanoic acids. Preferably, the enantiopure *(R)*-hydroxy-acylthioalkanoic acids are selected from the list comprising: 3-hydroxy-6-acetylthiohexanoic, 3-hydroxy-4-acetylthiobutanoic, and hydroxylated derivatives of 6-acetylthiohexanoic acid, 5-acetylthiopentanoic acid, 2,5-bis (acetylsulfanyl) pentanoic acid, 4-acetylthiolbutanoic acid, 3-acetylsulfanylbutanoic acid, 11-acetylthioundecanoic acid, 16-acetylthiolhexadecanoic acid, 5-acetylthio-octanoic acid, 5-propane-ylthio-octanoic acid, 5-butane-ylthio-octanoic acid, 12-acetyl-thio-dodecanoic acid, 6-acetylsulfanyl-8-methylsulfanyl-octanoic acid, 8-acetylsulfanyl-6- sulfanyloctanoic acid, 5-(2-methylpropane-ylsulfanyl) octanoic acid, 6,8-bis(acetylsulfanyl)octanoic acid.

The term "enantiopure" as used herein, relates to compounds, in this case *(R)*-3-hydroxy-acylthioalkanoic acids, only with a specific enantiomeric form.

Hydroxylated derivatives of *(R)*-3-hydroxy-acylthioalkanoic acids are those acids that have at least one hydroxyl group.

A sixth aspect of the invention relates to a process for the production of enantiopure *(R)*-3-hydroxy-acylthioalkanoic acids comprising the hydrolysis of the PHACOS. Hydrolysis of PHACOS can be performed by any of state-of-the-art processes known. Preferably, chemical hydrolysis. Preferably, enzymatic hydrolysis. More preferably hydrolysis is performed by a depolymerase enzyme. Preferably, the enzyme is depolymerase PhaZ. The depolymerase PhaZ is able to hydrolyze PHA granules containing both aliphatic and aromatic monomers (de Eugenio et al., 2007. The Journal of Biological Chemistry. 282, 7: 4951-62).

A seventh aspect of the invention relates to enantiopure *(R)*-3-hydroxy-acylthioalkanoic acids obtained following the procedure outlined in the fifth aspect of the invention. These compounds are useful in the pharmaceutical and/or chemical industry for use as synthons.

One embodiment of the invention is a PHA synthesis procedure whereby *P*. *putida* KT42FadB is grown in a culture medium with 2.4 mM decanoic acid as inducer and 12 mM 6-acetylthiohexanoic acid as functionalized compound, in two-stage fermentation, with the second stage lasting 24 hours.

Throughout the description and the claims, the word "comprise" and its variations are not intended to exclude other technical characteristics, additives, components or steps. For persons skilled in the art, other uses, advantages and characteristics of this invention will be apparent, partly from the description and partly from putting this invention into practice. The following examples and figures are provided by way of illustration, and are not considered limitative of the present invention.

### DESCRIPTION OF THE FIGURES

**Figure 1****.** Diagram showing the construction of the *P. putida* KT42FadB strain using the pK18*mob* plasmid. The plasmid has the lacZ gene promoter (Plac), a multicloning site (MCS), the alpha-*lac*Z gene and a kanamycin resistance gene (km^{R}). Part of the *fadB* gene is inserted between the *Smal* restriction sites. The *Sphl* restriction enzyme provides information about the orientation of the insert. Homologous recombination allows insertion of the plasmid within gene *fadB* of *P. putida* KT2442 in order to generate the KT42FadB strain.
**Figure 2****.** *P. putida* KT2442 and *P. putida* KT42FadB grown on 0.1 N M63 medium agar plates with 15 mM sodium octanoate. The original strain grows but the mutant strain is unable to grow in this medium.
**Figure 3****.** Growth curves of *P*. *putida* KT2442 and KT42FadB strains in the presence of 12mM decanoic acid in a two-stage fermentation strategy.
**Figure 4****.** PHA content and monomeric composition of *P*. *putida* KT2442 (KT) and KT42FadB (FadB) cultures growing in the presence of 12mM decanoic acid in a two-stage fermentation strategy in a liquid medium at different times (5, 10, 15, 20, 25, 30 and 90 hours). OH-C6 is hydroxyhexanoic acid, OH-C8 is hydroxyoctanoic acid. OH-C10 is hydroxydecanoic acid.
**Figure 5****.** Growth curves of *P*. *putida* KT2442 and KT42FadB strains grown in a two-stage fermentation procedure with: 15 mM 6-ATH as sole carbon source (Δ); 12mM 6-ATH as carbon source and 2.4 mM decanoic acid as inducer (○); 2.4 mM decanoic acid at the induction concentration (□).
**Figure 6****.** GC-MS analysis of methyl esters of 3-hydroxyhexanoate (peak 1), 3-hydroxyoctanoate (peak 3), 3-hydroxydecanoate (peak 4), and 3-hydroxy-6-acetylthiohexanoate (peak 5) in a *P. putida* KT42FadB using as carbon source: (A) 12 mM decanoic acid and (B) 12 mM 6-ATH as carbon source plus 2.4 mM decanoic acid as inducer. Peak 2 corresponds to3-methylbenzoate, used as internal standard.
**Figure 7****.** 1H NMR spectrum of the polymer isolated from the *P. putida* KT2442 strain.
**Figure 8****.** 1 H NMR spectrum of the polymer isolated from the *P*. *Putida* KT42FadB strain.

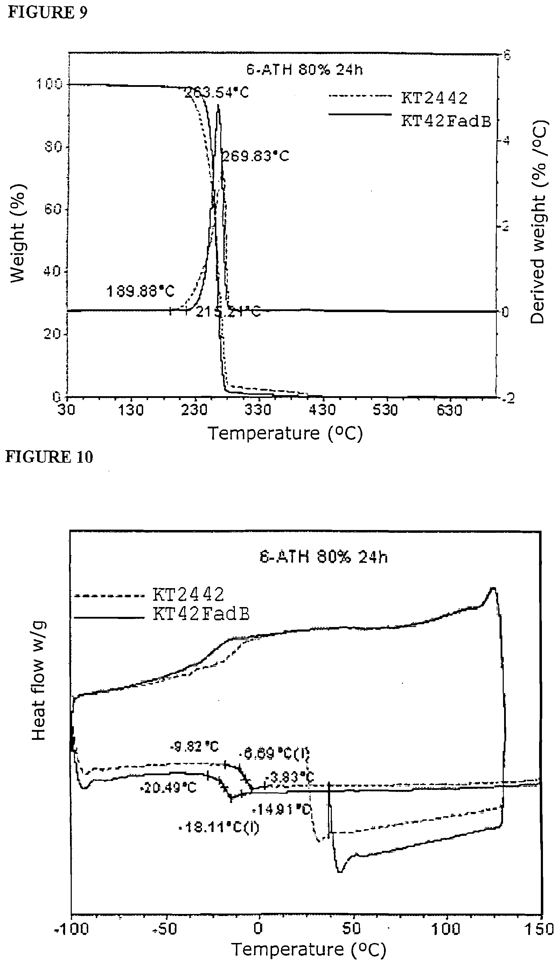

**Figure 9****.** TGA thermogram of polymer samples isolated from *P. putida* KT2442 and *P. putida* KT42FadB strains. It represents weight % versus temperature.
**Figure 10****.** TGA thermogram of polymer samples isolated from *P*. *putida* KT2442 and *P. putida* KT42FadB strains. It represents the heat flow versus temperature.

### EXAMPLES

The PHA synthesis process of the present invention is illustrated below.

### EXAMPLE 1: Study of the phenotype of the P. putida KT42FadB mutant strain in terms of its ability to metabolize fatty acids and convert them into PHA using aliphatic precursors.

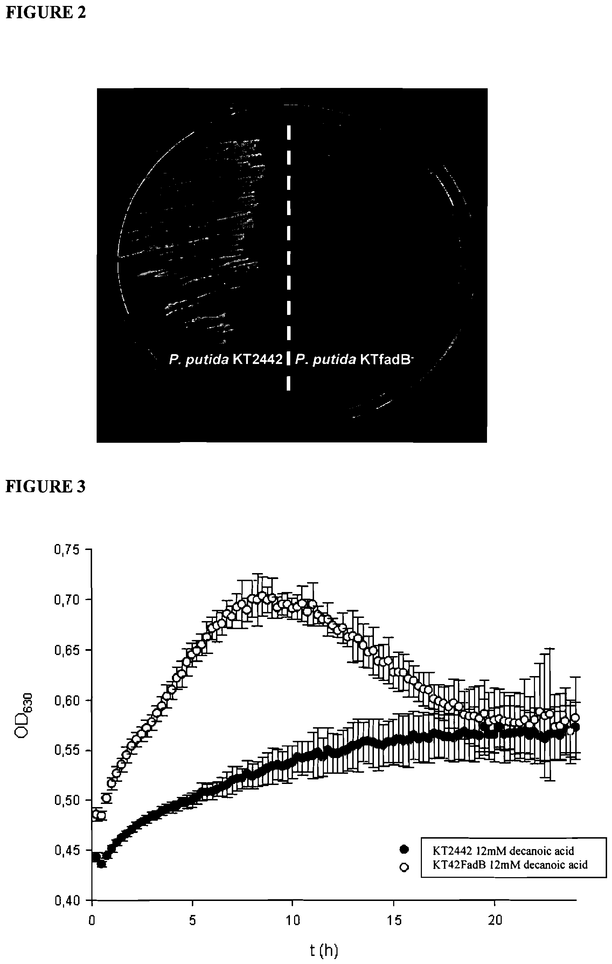

To analyse the phenotype of the *P. putida* KT42FadB mutant strain in terms of its ability to use fatty acids as carbon source, this strain was first grown in plates containing optimal medium for PHA production in *P. putida* KT2442 (0.1 N M63 medium with 15 mM octanoic acid). As shown in Figure 2, *P*. *putida* KT42FadB was unable to grow on agar plates with this medium, since the gene *fadB* mutation prevents it from using octanoic acid as carbon and energy source due to the deficiency in production of the FadAB enzyme complex. This confirmed the phenotype corresponding to the disruption of the fatty acid beta-oxidation pathway in this mutant.

We have also studied the growth and PHA production capacity using PHA precursors unrelated to fatty acids in *P. putida* KT42FadB strain, compared with the wild-type strain. Both *P. putida* strains show similar growth profiles and are able to form PHA granules when grown in a one-stage fermentation process. Using either rich medium like LB or nitrogen-limited minimal media with various carbon sources, such as 0.1 N M63, 20 mM glucose; or 0.1 N M63, 30 mM succinate, no apparent differences were observed between *P*. *putida* wild-type type and KT42FadB mutant strains in terms of PHA production, which was low in all cases (15-20% dry weight). These results are consistent with a mutation in the beta-oxidation complex which affects mutant strain growth only when fatty acids are used as carbon source, but not when the cells are fermented with carbon sources other than fatty acids.

### STRAINS, MEDIA AND CULTURE CONDITIONS

Table 1 shows the bacterial strains used in this study. The *E. coli* and *P*. *putida* strains were grown in LB medium at 37 °C and 30 °C, respectively. Kanamycin was used as selection antibiotic (50 µg/ml). The *P. putida* strains were also grown in 0.1 N M63 (13.6 g/L KH₂PO₄, 0,2 g/l (NH₄)₂SO₄, 0,5 mg/l FeSO₄·7 H₂O, adjusted to pH 7.0 with KOH), a nitrogen-limited minimal medium, at a temperature of 30 °C and shaking at 250 rpm as previously described (Moldes et al., 2004. Appl. Environ. Microbiol. 70, 3205). This medium was supplemented with 1 mM MgSO₄ and trace elements solution (composition x 1,000: 2.78 g/L FeSO₄·7H₂O, 1.98 g/L MnCl₂·4H₂O, 2.81 g/L CoSO₄·7H₂O, 1.47 g/L CaCl₂·2H₂O, 0.17 g/L CuCl₂·2H₂O, 0.29 g/l ZnSO₄·7H₂O). Carbon sources were: 20 mM glucose, 30 mM succinate, 15 mM sodium octanoate, 12 mM decanoic acid and 15 mM 6-acetylthiohexanoic acid (6-ATH), or mixtures of these (in the ratio indicated in each experiment). Growth was monitored with a Shimadzu UV-260 spectrophotometer at 600 nm. Solid media were supplemented with 1.5% (wt/vol) of agar.

**Table 1. Bacterial strains used.**

| **Strains** | **Origin or Use** |
|---|---|
| *Pseudomonas putida* KT2442 | *hsd*MR. Derivative strain of the parental strain KT2440 |
| *Pseudomonas putida* KT42FadB | *ΔfadB*::pK18*mob* |
| *Escherichia coli* DH10B | Host for plasmid construction and as a vector donor via conjugation |
| *Escherichia coli* HB101 (pRK600) | Helper strain for triparental mating |

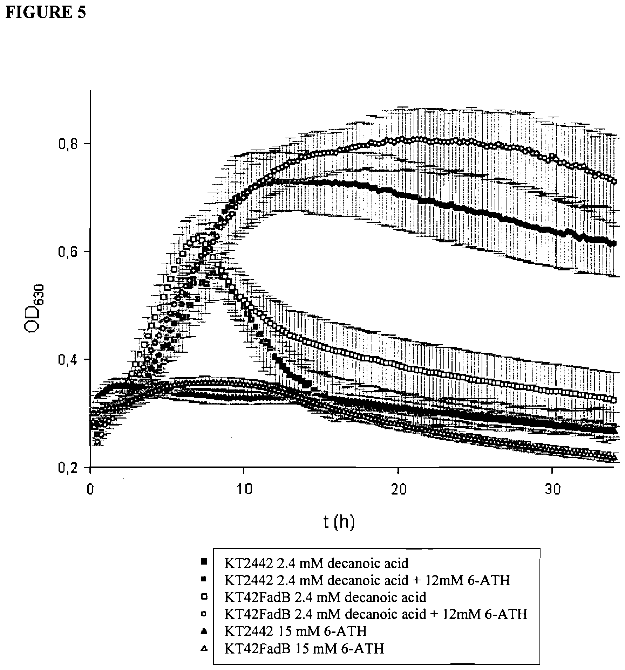

*P. putida* KT2442 and the mutant strain KT42FadB were also grown in a two-stage process. Each of these strains pre-inoculated in LB medium were adjusted to an optical density at 600 nm (OD₆₀₀) of 0.3. After 16 hours of incubation in LB, the cells were collected and diluted 1:2 in 0.1 N M63 medium supplemented with the chosen carbon source. This second growth stage on minimal medium was also performed in 96- well plates. For this purpose, 200 µl aliquots of the said cultures were placed in multiwell plates. The plates were incubated at 30 °C for 24 to 48 hours, with 20 seconds of intense orbital shaking every 15 minutes. The growth curves show the average values of at least 10 repeats (Figures 3 and 5).

### DNA Manipulations and Plasmid Constructions

Genetic manipulations and other molecular biology techniques were essentially performed according to previous reports (Sambrook J. and Russell D.W., 2001. Cold Spring Harbor Laboratory Press, Cold Spring Harbor, NY.). To construct the mutant *P. putida* KT42FadB, a 707 bp internal fragment of the *fadB* gene was cloned into the pK18*mob* plasmid, using the primers: sense (SEQ ID NO: 1) 5' TCCCCCGGGAAAAGCTCAAGCTCAATGCCAT 3' and antisense (SEQ ID NO: 2) 5' TCCCCCGGGTTGAAGAAGTGCATGCC 3'. The resulting polymerase chain reaction (PCR) products and plasmid pK18mob (Schafer, A et al. 1994. Gene 145:69-73) were digested by *Smal* and then ligated. The ligation mixture was transformed into *E. coli* DH10B competent cells. Two plasmids were selected by alfa-complementation, differing in the orientation of *Plac* promoter versus the *fadB* gene internal fragment. The plasmid pK18FadB1, carrying the *Plac* promoter in the opposite orientation to the internal *fadB* fragment was inserted into *P. putida* KT2442 by triparental mating. Correct insertion of the pK18FadB1 plasmid in the *fadB* gene of the *P. putida* genome was confirmed by PCR amplification (Figure 1).

### Purification of PHA polymers

After 24 h of incubation, two-stage cultures in 0.1 N M63 were harvested by centrifugation and resuspended in salt solution. Cells were disrupted by passing them through a French pressure cell twice. These extracts were centrifuged for 30 min at 15,000xg, and the pellet fraction was dissolved in 10 ml of chloroform. To extract water-soluble cell components, 2 ml of water was added, and the organic and aqueous phases were mixed by vortexing and then separated by centrifugation at 5,000xg. The organic layer (lower) was transferred into a new tube. The procedure was repeated adding 10 ml of chloroform to the aqueous phase. The organic phase was precipitated in cold methanol (tenfold excess) under vigorous stirring with a magnetic bar. After decanting the methanol/chloroform mixture, the resulting polymer was resuspended in chloroform and air-dried overnight. The resulting polymers were stored at room temperature.

### EXAMPLE 2: PHA production in the mutant strain by a two-stage fermentation strategy.

The *P. putida* KT42FadB mutant strain is unable to utilize fatty acids as sole carbon source, and therefore cannot efficiently produce PHA from these precursors under a one-stage culture process in liquid medium. The wild-type strain produces 1 mg/ml of biomass when grown in 12 mM decanoic acid as growth source and PHA precursor, while the KT42FadB mutant produced only 0.34 mg/ml of cellular biomass.

We have developed a strategy consisting of two fermentation stages in order to compare the PHA-producing ability of the mutant and wild-type strain. In a first stage, the cells were cultured overnight in LB rich medium. After harvesting cells by centrifugation, they were diluted 1:2 times in fresh 0.1 N M63 medium supplemented with 12 mM decanoic acid as PHA precursor. This second stage, or PHA accumulation stage, we performed in multiwell plates in order to comparatively analyse the OD₆₃₀ of the cultures over time. After 7 to 8 hours of incubation in multiwell plates, optical density of the mutant strain KT42FadB cultures significantly increased compared with that of wild-type strain (Figure 3). Seeing that cell turbidimetry is altered by the accumulation of PHA granules (Eugenio et al., 2010. Environ. Microbiol. 12: 207-21), these results imply that there was an increase in PHA production in the mutant strain compared to the wild-type strain.

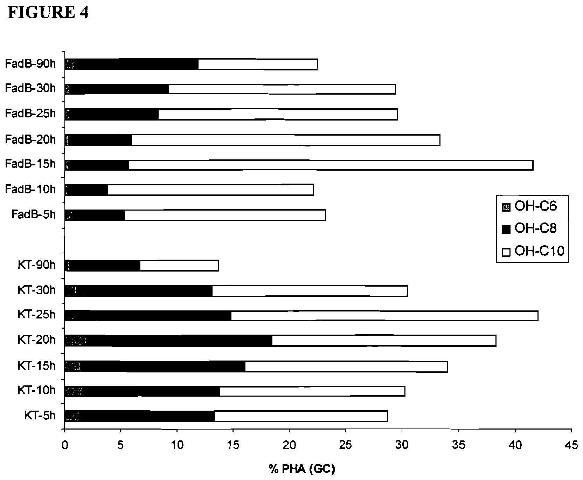

Cellular PHA content and composition were determined by gas chromatography-mass spectrometry (GC-MS) in dry sediment (dehydrated), obtained from *P.putida* KT2442 and KT42FadB cells after the second fermentation stage. When 12 mM decanoic acid is used as PHA precursor, the *P. putida* KT42FadB strain is able to accumulate higher PHA concentrations than the wild-type strain and with a different monomeric composition (Figure 4). The lack of the FadAB enzyme complex leads to an accumulation of (R)-3-hydroxyacyl-CoA metabolic intermediates within the cell, which are channelled to PHA production rather than following the beta-oxidation catabolic pathway. The PHA produced in the mutant strain contains a small proportion of monomers with short side chains, i.e., which have undergone a beta-oxidation catabolic process, suggesting the presence of alternative beta-oxidation pathways encoded in the *P. putida* KT2442 genome.

### Example 3: Accumulation of PHACOS in P. putida KT42FadB and in the wild-type strain from precursors functionalized with thioester groups.

The synthesis of PHA functionalized with thioester groups in the side chain (PHACOS) was compared in wild-type and KT42FadB mutant strains in terms of DO₆₃₀ when grown in multiwell plates. 6-acetylthiohexanoic acid (6-ATH) was chosen as functionalized precursor due to the reactivity of the thioester group in the side chain, as revealed by the chemical structures identified in PHACOS polyesters described in detail below. The optimum growth conditions for functionalized PHA production were established with decanoic acid as co-substrate (12 mM 6-ATH as carbon source and 2.4 mM decanoic acid as inducer) in a two stage fermentation culture. Under these conditions, the KT42FadB mutant showed higher turbidimetry values after 24 hours, compared to those detected in the wild-type strain (Figure 5). When decanoic acid was used as sole carbon source at a co-substrate concentration of 2.4 mM, the OD₆₃₀ increased significantly until 7-8 h of the second stage, and subsequently decreased. The turbidimetric profiles differed totally when cells were cultured with 12 mM 6-ATH plus 2.4 mM decanoic acid compared to exclusively 2.4 mM decanoic acid. These results demonstrate that *P*. *putida* KT2442, and to a greater extent *P*. *putida* KT42FadB, are able to metabolize 6-ATH molecules, but only in the presence of a co-substrate such as decanoic acid.

We analysed biomass and PHACOS production in both strains after 24 hours of the accumulation stage with 12 mM 6-ATH and 2.4 mM decanoic acid. Under these conditions, a similar biomass was attained by both strains: 0.9 mg/ml in the wild-type and 1.2 mg/ml in the KT42FadB mutant strain. Wild-type and mutant strains produced, respectively, 0.05 mg/ml and 0.22 mg/ml of PHACOS in relation to dry weight. When bacteria were grown in one growth stage with 12 mM 6-ATH and 2.4 mM decanoic acid, the biomass of wild-type and mutant strains was 0.65 mg/ml and 0.37 mg/ml, respectively. The wild-type strain and the mutant strain produced 0.09 mg/ml and 0.11 mg/ml of PHACOS, respectively.

### EXAMPLE 4: Composition of PHACOS polymers.

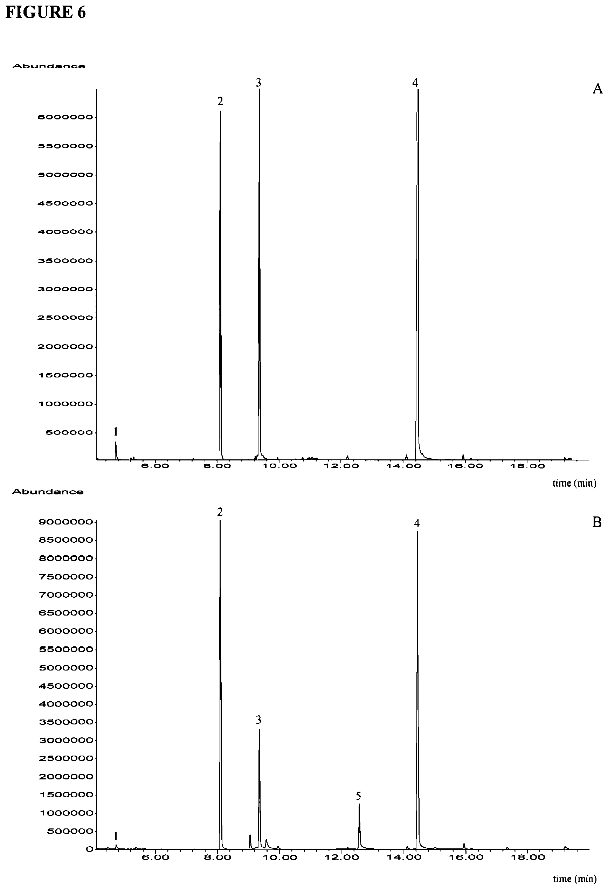

Figure 6 shows the total ions by GC-MS of each methyl ester obtained from the polymers of the *P. putida* KT42FadB bacteria when grown in two stages. As shown in the figure, each polymer contains 3-hydroxy-alkanoic acid (octanoic, decanoic and hexanoic traces), as well as monomers derived from 6-ATH when it is used as a precursor for bacterial growth.

Quantification of 3-hydroxy-alkanoic monomers was based on the signals obtained between standards, consisting of the respective fatty acids without the hydroxyl group, and the internal standard used in this study, 3-methyl-benzoate. Quantification of 3-hydroxy-6-acetyl-thiohexanoic methyl ester was performed with 6-ATH as standard. The concentration of each monomer was obtained by calculating the relative response factor, (RRF) which is a ratio of the area obtained for each compound and for the internal standard from a chromatogram, as well as the concentration of the standard and the internal standard used. Bacteria are known to be able to convert part of decanoic acid into shorter fatty acids (octanoic and hexanoic acids) as a step prior to their incorporation into the polymer. Similarly, it is thought that the bacteria might cut the 6-ATH side chain to incorporate a monomer with two carbons less in the polymer. Due to the lack of standards for 6-ATH-derived hydroxylated monomers, their proportion in the polymer was accurately determined by nuclear magnetic resonance (NMR).

It is worth highlighting that NMR cannot be applied for rapid structural determination of the non functionalized PHA monomers, as the chemical shift referencing is so complex that chemical shifts in the monomers present in the polyester cannot be easily determined. For this reason, both techniques, GC-MS and NMR, have been used in a complementary way to identify and quantify each monomer present in the polymers.

**Table 2. Chemical shift in parts per million (ppm) of all monomers in the PHA, analysed by NMR 1 H.**

| **Shift (ppm)** | **Protons** |
|---|---|
| 0.8 | A |
| 1.3 | G |
| 1.6 | h +h₁ |
| 1.7 | d₁ |
| 2.3 | b₁ +b₂ |
| 2.5 | E |
| 2.8 | c₁ |
| 3.2-3.3 | h₂ |
| 5.2 | F |
| 5.3 | f₂ |

The chemical structures identified in PHACOS polyesters are the following:

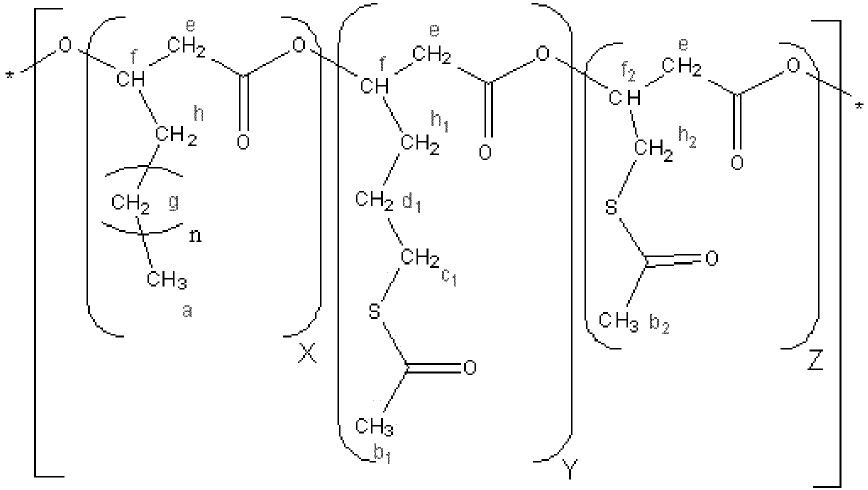

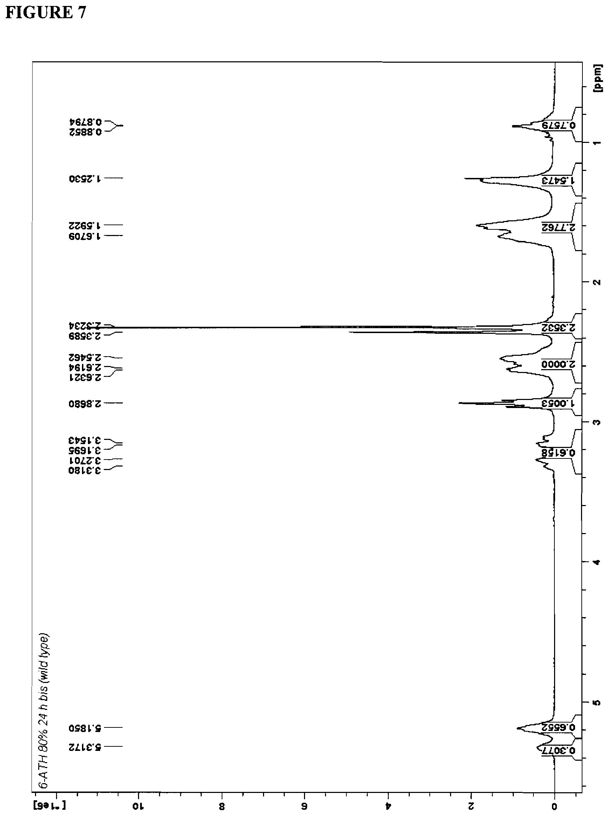

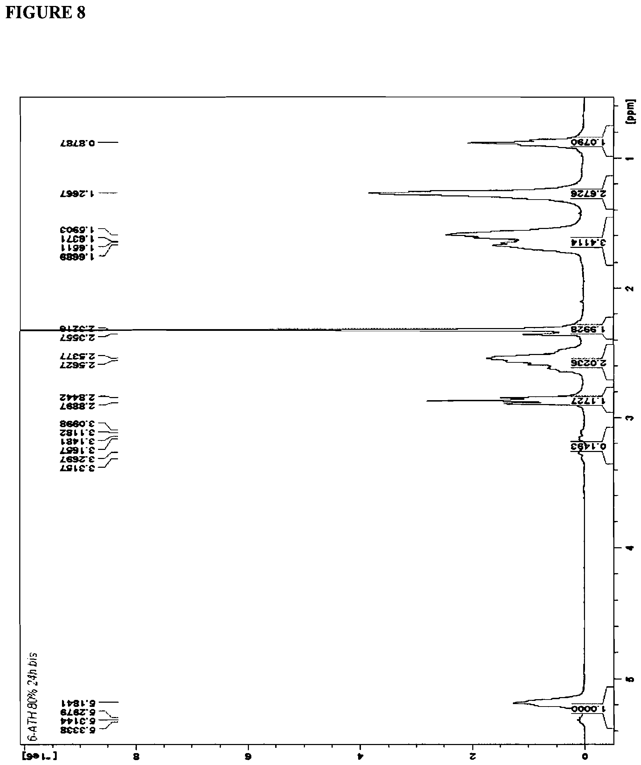

Figures 7 and 8 show the NMR spectra of PHACOS produced by the mutant strain and wild-type strain, while Table 2 shows the chemical shifts of the protons indicated (a, b₁, b₂, etc.) in the chemical structures shown above.

### Determination of monomeric composition

It should be noted that the polymer obtained from the mutant bacteria has a higher percentage of 3-hydroxy-6-ATH than that obtained from the wild-type strain. The wild-type bacteria also have a greater ability to metabolize the 6-ATH molecule, thus a smaller proportion of the 3-hydroxy-4-acetylthiobutanoic monomer is observed. Table 3 summarises the monomeric composition of PHACOS obtained from the mutant and the wild-type strains when cultured in two stages. GC-MS and NMR were used to determine the monomeric composition of the PHAs.

**Table 3. Monomeric composition in % of the polymers isolated from P. putida KT2442 and KT42FadB strains**

| **Monomer** | **KT2442** | **KT42FadB** |
|---|---|---|
| 3-hydroxy-6-acetylthiohexanoate (%) | 47 | 57.5 |
| 3-hydroxy-4-acetylthiobutanoate (%) | 31.5 | 7.5 |
| 3-hydroxy-decanoate | 7.1 | 19.1 |
| 3-hydroxy-octanoate | 12.6 | 15.5 |
| 3-hydroxy-hexanoate | 2.1 | 0.6 |

For GC-MS analysis, monomers were obtained from the polyester by acid methanolysis or by suspending 3-6 mg of freeze-dried whole cells in 2 ml of methanol acidified with 15% (v/v) H₂SO₄ containing 0.5 mg/ml of 3-methyl-benzoate, used as internal standard, and 2 ml of chloroform. The mixture was placed in screw-cap tubes and then incubated at 100 °C for 4 hours. After cooling, 1 ml of distilled water was added to the mixture to induce phase separation and the the organic layer containing the methyl esters of the resulting monomers were analysed by GC-MS. The equipment used for this analysis was an Agilent 7890A gas chromatographer (GC) equipped with a HP 5 MS column (30 m, 0.25 mm internal diameter and 0.25 mm film thickness) and a mass spectrometer (MS) Agilent 5975C. A sample of 1µl in the organic phase was injected (with split ratio 1:50) with helium as carrier gas and oven temperature was programmed to remain at 80 °C for 2 minutes and then increase from 15 °C up to 250 °C for efficient separation of the peaks. The injector temperature was 250 °C. The spectra were obtained as electron impact with MS operating ionization energy of 70 eV.

Nuclear magnetic resonance (NMR) was performed on a Bruker 400 MHz apparatus with a sample solution in CDCl₃ (20 mg/ml deuterated chloroform) to determine the relationship between each monomer.

### Example 5: Physical and thermal characterization of PHACOS polymers.

According to the results of thermogravimetric analysis (TGA, air atmosphere, 10 °C/ minute), the thermograms confirmed that all samples have only organic matter as neither water nor inorganic waste were detected. The weight remained stable up to 200 °C for the PHACOS sample produced by the wild-type strain and up to 220 °C for the PHACOS sample produced by the mutant strain. Above these temperatures polymer degradation occurred, with a maximum rate centred at 270 °C for the sample corresponding to the wild-type strain and at 264 °C for the sample corresponding to the mutant strain. The unburnt residues detected at 700 °C were of very low concentration and similar between samples.

The Differential Scanning Calorimetry (DSC) analyses showed that both polyesters are amorphous. There was a continuous glass transition temperature (Tg) of -7 °C and -18 °C for samples of wild-type and mutant strains, respectively. No melting or crystallization peaks could be detected during sample heating and cooling at 10 °C/min.

According to the thermal characterization of the polyesters studied in this work, both exhibit low glass transition temperature (Tg) values. This could indicate good thermal processing capacity as low Tg values suggest that both polymers behave like amorphous polymers and are relatively soft and deformable at room temperature.

**Table 4. Molecular weights and polydispersity index (PDI) of polymers isolated from P. putida KT2442 and KT42FadB strains.**

| | KT2442 | KT42FadB |
|---|---|---|
| **Mₙ (kDa)** | 71 | 99 |
| **M_{w} (kDa)** | 259 | 366 |
| **PDI** | 3.6 | 3.7 |

The results are shown in Table 4 with respect to the determination of molecular weight. Results show that the sample from mutant KT42FadB has higher average molecular weight values both for number (Mₙ) and weight (M_{w}), which indicates that the polymerization process is not inhibited by the addition of monomers with the thioester group to the polyester.

Regarding the polydispersity index (PDI), this is also higher in the mutant strain, indicating a greater degree of variation in the molecular weights of this polymer.

According to the results obtained, we can say that we have synthesized two new hydrophilic PHAs with functional groups that are highly susceptible to being chemically modified and with thermal characteristics that suggest great ease of processing.

### Physical and thermal characterization

The molecular weights of the polyesters were determined by size exclusion chromatography (SEC). Measurements were taken using a Shimadzu liquid chromatographer (Japan) equipped with a RID-10A refractive index detector and a SPD-M10A diode array UV detector. The columns set used was composed of a 50 mm PLgel Guard 5 µm column, two 300 PLgelMIXED-C 5 µm columns, and a 300 mm PLgel 5 µm-100 Å column. The calibration curve was performed with polystyrene standards from 580 to 1.6 106 g/mol. Chloroform was used as the mobile phase and the analyses were carried out at 25 °C with a solvent flow rate of 0.8 ml min.

Thermogravimetric analysis (TGA) was performed on a TA Instruments TGA Q5000 (USA). Samples were heated from room temperature up to 700 °C at 10 °C/min in air. TGA provides a quantitative measurement of the variation in the mass of polyesters associated with transition and thermal degradation.

Differential scanning calorimetry (DSC) measurements were conducted on a TA Instruments DSC 2910. The materials were exposed to successive thermal cycles of heat-cool-heat, between -90 °C and 180 °C at 10 °C/min. DSC was used to determine the polymers' thermal properties such as glass transition temperature (Tg), crystallization temperature (Tc), and melting temperature (Tm).

### Example 6: Production of PHACOS in other species.

The production of PHA with thioester groups in the side chain was carried out in two stages in 0.1 N M63 plus 12 mM 6-ATH and 2.4 mM decanoic acid using other strains of *Pseudomonas* such as *P. putida* U (0.02 mg/ml of PHACOS), *P. putida* U ΔFadBA (mutant *fadBA⁻)* (0.2 mg/ml of PHACOS) and *P. oleovorans* GPo1 (0.04 mg/ml of PHACOS). PHA granule production was detected in all cases (Table 5).

**Table 5. Monomeric composition in % of the polymers isolated from different strains of P. putida KT2442.**

| | **% aliphatic monomers** | **% thiol monomers** |
|---|---|---|
| *P. putida U* | >95% | Traces |
| *P. putida* U FadBA- | 83.50% | 16.50% |
| *P. oleovorans* GPo1 | >95% | Traces |

## Claims

1. A process for the synthesis of polyhydroxyalkanoates (PHAs) comprising monomers containing thioester groups in the side chain that comprises culturing a cell capable of producing PHA which is a bacterium of the genus *Pseudomonas* in a substrate, wherein the said substrate comprises at least one acylthioalkanoic acid and at least a fatty acid of between 4 and 28 carbons not functionalized with a thioesther group.

2. The process according to claim 1 is **characterised in that** the bacterium of the genus *Pseudomonas* is selected from the list that comprises: *P. putida* KT2442; *P. putida* U; *P. oleovorans* GPo1; *P. putida* KT42FadB with accession number CECT 7772 and *P. putida* U ΔFadBA, preferably *P. putida* KT42FadB with accession number CECT 7772.

3. The process according to any of claims 1 or 2 **characterised in that** the acylthioalkanoic acid is selected from the list that comprises the following acids: 6-acetylthiohexanoic acid, 5-acetylthiopentanoic acid; 2,5-bis(acetylsulfanyl)pentanoic acid; 4-acetylthiolbutanoic acid; 3-acetylsulfanylbutanoic acid; 11-acetylthioundecanoic acid; 16-acetylthiolhexadecanoic acid; 5-acetylthio-octanoic acid; 5-propane-ylthio-octanoic acid; 5-butane-ylthio-octanoic acid; 12-acetylthio-dodecanoic acid; 6-acetylsulfanyl-8-methylsulfanyl-octanoic acid; 8-acetylsulfanyl-6-sulfanyloctanoic acid: 5-(2 -methylpropane-yl sulfanyl) octanoic acid; 6,8-bis (acetylsulfanyl)octanoic acid, preferably 6-acetylthiohexanoic acid.

4. The process according to any of claims 1 to 3 **characterised in that** the fatty acid of between 4 and 28 carbons is selected from the list comprising: decanoic acid, nonanoic acid and octanoic acid, preferably is decanoic acid.

5. The process according to any of claims 1 to 4 **characterised in that** the substrate comprises decanoic acid and 6-acetylthiohexanoic acid.

6. The process according to any of claims 4 or 5 **characterised in that** the substrate comprises a molar ratio of fatty acid of between 4 and 28 carbons and acylthioalkanoic acid of between 1:9 and 9:1, preferably 1.1:8.9 and 2:8, more preferably 1.5:8.5 and 1.8:8.2.

7. The process according to claim 6 **characterised in that** the substrate consists of 2.4 mM decanoic acid and 12 mM 6-acetylthiohexanoic acid.

8. The process according to any of claims 1 to 7 **characterised in that** culture comprises one or two fermentation stages (one- or two-stage culture).

9. The process according to claim 8 **characterised in that** the second fermentation stage lasts between 5 and 30 hours, preferably, between 7 and 26 hours, more preferably between 8 and 25 hours.

10. PHAs obtained by the process according to any of claims 1 to 9, which comprise monomers containing thioester groups in the side chain.

11. Bacterial strain of the species *Pseudomonas putida* with accession number CECT 7772.

12. Use of PHAs according to claim 10 to produce bioplastic materials, fabrics, biomaterials, drug delivery systems, adhesives or paints.

13. Use of PHAs according to claim 10 to obtain enantiopure (R)-3-hydroxy-acylthioalkanoic acids, wherein preferably enantiopure (R)-3-hydroxy-acylthioalkanoic acids are selected from the list that comprises: 3-hydroxy-6-acetylthiohexanoic acid; 3-hydroxy-4-acetylthiobutanoic acid and hydroxylated derivatives of 5-acetylthiopentanoic acid; 2,5-bis(acetylsulfanyl) pentanoic acid; 4-acetylthiolbutanoic acid; 3-acetylsulfanylbutanoic acid; 11-acetylthioundecanoic acid; 16-acetylthiolhexadecanoic acid; 5-acetylthio-octanoic acid; 5-propane-ylthio-octanoic acid; 5-butane-ylthio-octanoic acid; 12-acetyl-thio-dodecanoic acid; 6-acetylsulfanyl- 8-methylsulfanyl-octanoic acid; 8-acetylsulfanyl-6-sulfanyloctanoic acid; 5-(2-methylpropane-ylsulfanyl) octanoic acid; 6,8-bis(acetylsulfanyl) octanoic acid.

14. A process for the production of enantiopure (R)-3-hydroxy-acylthioalkanoic acids that comprises hydrolysis of PHAs according to claim 10, wherein the hydrolysis involves chemical or enzymatic hydrolysis, preferably the enzymatic hydrolysis is performed by a depolymerase enzyme, more preferably the depolymerase enzyme is PhaZ.

15. Enantiopure (R)-3-hydroxy-acylthioalkanoic acids obtained by the process according to claim 14.

## Patentansprüche

1. Verfahren zur Synthese von Polyhydroxyalkanoaten (PHA) mit Thioestergruppen in der Seitenkette enthaltenden Monomeren, das das Züchten einer Zelle, die zur Herstellung von PHA in der Lage ist und eine Bakterie der Gattung *Pseudomonas* darstellt, in einem Substrat umfasst, wobei das besagte Substrat mindestens eine Acylthioalkansäure und mindestens eine Fettsäure mit 4 bis 28 Kohlenstoffen, die nicht mit einer Thioestergruppe funktionalisiert sind, umfasst.

2. Verfahren nach Anspruch 1, **dadurch gekennzeichnet, dass** die Bakterie der Gattung *Pseudomonas* ausgewählt wird aus der Liste bestehend aus: *P*. *putida* KT2442; *P. putida* U; *P. oleovorans* GPo1; *P. putida* KT42FadB mit der Zugangsnummer CECT 7772 und *P. putida* U ΔFadBA, vorzugsweise *P. putida* KT42FadB mit der Zugangsnummer CECT 7772.

3. Verfahren nach einem der Ansprüche 1 oder 2, **dadurch gekennzeichnet, dass** die Acylthioalkansäure ausgewählt wird aus der Liste, die folgende Säuren umfasst: 6-Acetylthiohexansäure; 5-Acetylthiopentansäure; 2,5-Bis(acetylsulfanyl)pentansäure; 4-Acetylthiobutansäure; 3-Acetylsulfanylbutansäure; 11-Acetylthioundecansäure; 16-Acetylthiohexadecansäure; 5-Acetylthio-octansäure; 5-Propanylthio-octansäure; 5-Butanylthio-octansäure; 12-Acetylthiododecansäure; 6-Acetylsulfanyl-8-methylsulfanyloctansäure; 8-Acetylsulfanyl-6-sulfanyloctansäure; 5-(2-Methylpropanylsulfanyl)-octansäure; 6,8-Bis(acetylsulfanyl)octansäure, vorzugsweise 6-Acetylthiohexansäure.

4. Verfahren nach einem der Ansprüche 1 bis 3, **dadurch gekennzeichnet, dass** die Fettsäure mit 4 bis 28 Kohlenstoffen ausgewählt wird aus der Liste bestehend aus: Decansäure, Nonansäure und Octansäure, wobei Decansäure bevorzugt ist.

5. Verfahren nach einem der Ansprüche 1 bis 4, **dadurch gekennzeichnet, dass** das Substrat Decansäure und 6-Acetylthiohexansäure umfasst.

6. Verfahren nach einem der Ansprüche 4 oder 5, **dadurch gekennzeichnet, dass** das Substrat ein molares Verhältnis von Fettsäure mit 4 bis 28 Kohlenstoffen zu Acylthioalkansäure von 1:9 bis 9:1, vorzugsweise von 1,1:8,9 bis 2:8, besonders bevorzugt von 1,5:8,5 bis 1,8:8,2 umfasst.

7. Verfahren nach Anspruch 6, **dadurch gekennzeichnet, dass** das Substrat aus 2,4 mM Decansäure und 12 mM 6-Acetylthiohexansäure besteht.

8. Verfahren nach einem der Ansprüche 1 bis 7, **dadurch gekennzeichnet, dass** die Kultur eine oder zwei Gärungsstufen (ein- oder zwei-Stufen-Kultur) umfasst.

9. Verfahren nach Anspruch 8, **dadurch gekennzeichnet, dass** die zweite Gärungsstufe 5 bis 30 Stunden, vorzugsweise 7 bis 26 Stunden, besonders bevorzugt 8 bis 25 Stunden in Anspruch nimmt.

10. Mit dem Verfahren nach einem der Ansprüche 1 bis 9 erhaltene PHA, die Monomere mit Thioestergruppen in der Seitenkette umfassen.

11. Bakterienstamm der Art *Pseudomonas putida* mit der Zugangsnummer CECT 7772.

12. Verwendung der PHA nach Anspruch 10 zur Herstellung von bioplastischen Materialien, Geweben, Biomaterialien, Arzneimittelfreisetzungssystemen, Klebern oder Lacken.

13. Verwendung der PHA nach Anspruch 10 zur Gewinnung von enantiomerenreinen (R)-3-Hydroxyacylthioalkansäuren, wobei die enantiomerenreinen (R)-3-Hydroxyacylthioalkansäuren vorzugsweise ausgewählt werden aus der Liste bestehend aus: 3-Hydroxy-6-acetylthiohexansäure; 3-Hydroxy-4-acetylthiobutansäure und hydroxylierte Derivate der 5-Acetylthiopentansäure; 2,5-Bis(acetylsulfanyl)pentansäure; 4-Acetylthiobutansäure; 3-Acetylsulfanylbutansäure; 11-Acetylthioundecansäure; 16-Acetylthiohexadecansäure; 5-Acetylthio-octansäure; 5-Propanylthio-octansäure; 5-Butanylthio-octansäure; 12-Acetylthiododecansäure; 6-Acetylsulfanyl-8-methylsulfanyloctansäure; 8-Acetylsulfanyl-6-sulfanyloctansäure; 5-(2-Methylpropanylsulfanyl)-octansäure; 6,8-Bis(acetylsulfanyl)octansäure.

14. Verfahren zur Herstellung von enantiomerenreinen (R)-3-Hydroxyacylthioalkansäuren, das die Hydrolyse der PHA nach Anspruch 10 umfasst, wobei die Hydrolyse eine chemische oder enzymatische Hydrolyse beinhaltet, wobei die enzymatische Hydrolyse vorzugsweise mittels eines Depolymerase-Enzyms durchgeführt wird und das Depolymerase-Enzym besonders bevorzugt PhaZ ist.

15. Enantiomerenreine (R)-3-Hydroxyacylthioalkansäuren, die mit dem Verfahren nach Anspruch 14 erhalten werden.

## Revendications

1. Procédé de synthèse des polyhydroxyalcanoates (PHA) comprenant des monomères contenant des groupes thioesters dans la chaîne latérale qui comprend la culture d'une cellule capable de produire le PHA qui est une bactérie du genre *Pseudomonas* dans un substrat, où ledit substrat comprend au moins un acide acylthioalcanoïque et au moins une acide gras de 4 à 28 carbones non fonctionnalisés avec un groupe thioesther.

2. Procédé selon la revendication 1 **caractérisé en ce que** la bactérie du genre *Pseudomonas* est sélectionnée dans la liste comprenant: *P. putida* KT2442; *P. putida U ; P. oleovorans* GPo1; *P. putida* KT42FadB sous le numéro d'accès CECT 7772 et *P. putida* U ΔFadBA, de préférence *P. putida* KT42FadB sous le numéro d'accès CECT 7772.

3. Procédé selon l'une quelconque des revendications 1 ou 2 **caractérisé en ce que** l'acide acylthioalcanoïque est sélectionné dans la liste comprenant les acides suivants: acide 6-acétylthiohexanoïque, acide 5-acétylthiopentanoïque; acide 2,5-bis(acétylsulfanyl)pentanoïque; acide 4-acétylthiolbutanoïque; acide 3-acétylsulfanylbutanoïque; acide 11-acétylthioundécanoïque; acide 16-acétylthiolhexadécanoïque; acide 5-acétylthio-octanoïque; acide 5-propane-ylthiooctanoïque; acide 5-butane-ylthio-octanoïque; acide 12-acétylthio-dodécanoïque; acide 6-acétylsulfanyl-8-méthylsulfanyl-octanoïque; acide 8-acétylsulfanyl-6-sulfanyloctanoïque: acide 5-(2 méthylpropane-yl sulfanyl) octanoïque; acide 6,8-bis (acétylsulfanyl)octanoïque, de préférence l'acide 6-acétylthiohexanoïque.

4. Procédé selon l'une quelconque des revendications 1 à 3 **caractérisé en ce que** l'acide gras comprenant entre 4 et 28 carbones est sélectionné dans la liste comprenant: l'acide décanoïque, l'acide nonanoïque et l'acide octanoïque, de préférence l'acide décanoïque.

5. Procédé selon l'une quelconque des revendications 1 à 4 **caractérisé en ce que** le substrat comprend l'acide décanoïque et l'acide 6-acétylthiohexanoïque.

6. Procédé selon l'une quelconque des revendications 4 ou 5 **caractérisé en ce que** le substrat comprend un rapport molaire d'acide gras compris entre 4 et 28 carbones et d'acide acylthioalcanoïque compris entre 1:9 et 9:1, de préférence entre 1.1 :8.9 et 2:8, plus préférentiellement entre 1.5:8.5 et 1.8:8.2.

7. Procédé selon la revendication 6 **caractérisé en ce que** le substrat contient 2.4 mM d'acide décanoïque et 12 mM d'acide 6-acétylthiohexanoïque.

8. Procédé selon l'une quelconque des revendications 1 à 7 **caractérisé en ce que** la culture comprend une ou deux étapes de fermentation (culture en une ou deux étapes).

9. Procédé selon la revendication 8 **caractérisé en ce que** la deuxième étape de fermentation a une durée comprise entre 5 et 30 heures, de préférence, entre 7 et 26 heures, plus préférentiellement entre 8 et 25 heures.

10. PHA obtenus avec le procédé selon l'une quelconque des revendications 1 à 9, ces derniers comprennent des monomères contenant des groupes de thioester dans la chaîne latérale.

11. Souche bactérienne de l'espèce *Pseudomonas putida* sous le numéro d'accès CECT 7772.

12. Utilisation des PHA selon la revendication 10 pour la production de matériaux bioplastiques, de tissus, de biomatériaux, de systèmes d'administration de médicaments, d'adhésifs ou de peintures.

13. Utilisation des PHA selon la revendication 10 pour l'obtention d'acides (R)-3-hydroxyacylthioalcanoïques énantiopurs, où, de préférence, les acides (R)-3-hydroxyacylthioalcanoïques énantiopurs sont sélectionnés dans la liste comprenant: l'acide 3-hydroxy-6-acétylthiohexanoïque; l'acide 3-hydroxy-4-acétylthiobutanoïque et des dérivés hydroxylés de l'acide 5-acétylthiopentanoïque; l'acide 2,5-bis(acétylsulfanyl) pentanoïque; l'acide 4-acétylthiolbutanoïque; l'acide 3-acétylsulfanylbutanoïque; l'acide 11-acétylthioundecanoïque; l'acide 16-acétylthiolhexadécanoïque; l'acide 5-acétylthio octanoïque; l'acide 5-propane-ylthio-octanoïque; l'acide 5-butane-ylthio-octanoïque; l'acide 12-acétyle-thio-dodécanoïque; l'acide 6-acétylsulfanyl-8-méthylsulfanyl-octanoïque; l'acide 8-acétylsulfanyl-6-sulfanyloctanoïque; l'acide 5-(2-méthylpropane-ylsulfanyl) octanoïque; l'acide 6,8-bis(acétylsulfanyl) octanoïque.

14. Procédé de production d'acides (R)-3-hydroxyacylthioalcanoïques énantiopurs qui comprend une hydrolyse des PHA selon la revendication 10, où l'hydrolyse implique une hydrolyse chimique ou enzymatique, de préférence l'hydrolyse enzymatique est obtenue par une enzyme dépolymérase, plus préférentiellement l'enzyme dépolymérase est PhaZ.

15. Acides (R)-3-hydroxy-acylthioalkanoïques énantiopurs obtenus avec le procédé selon la revendication 14.
